# EUROPEAN PATENT SPECIFICATION

(11) **EP 2 928 491 B1**
(45) Date of publication and mention of the grant of the patent: **21.11.2018**
(21) Application number: 13860917.7
(22) Date of filing: 03.12.2013
(51) Int. Cl.: A61K 39/104

(54) **FUSION PROTEINS FOR USE AS IMMUNOGENIC ENHANCERS FOR INDUCING ANTIGEN-SPECIFIC T CELL RESPONSES**
FUSIONSPROTEINE ZUR VERWENDUNG ALS IMMUNOGENE VERSTÄRKER ZUR INDUKTION VON ANTIGENSPEZIFISCHEN T-ZELL-ANTWORTEN
PROTÉINES DE FUSION DESTINÉES À ÊTRE UTILISÉES COMME ACTIVATEURS IMMUNOGÈNES POUR INDUIRE DES RÉPONSES EN CELLULES T SPÉCIFIQUES DE L'ANTIGÈNE

(30) Priority: 05.12.2012 US 201261733879 P
(43) Date of publication of application: 14.10.2015
(73) Proprietor: TheVax Genetics Vaccine Co., Ltd., Taipei City, Taiwan 10685 (CN)
(72) Inventor: CHOU, Wei-I, Zhubei City Hsinchu County Taiwan 302 (TW); WU, Chia-Mao, Zhubei City Hsinchu County Taiwan 302 (TW); WU, Jiun-Ming, Zhubei City Hsinchu County Taiwan 302 (TW); CHANG, Hsiu-Kang, Taipei City Taiwan 10685 (TW)
(74) Representative: Gill, Siân Victoria
(86) International application number: PCT/US2013/072804
(87) International publication number: WO 2014/089036

(56) References cited:
- EP-A1- 2 295 065
- EP-A2- 2 237 794
- WO-A2-00/61772
- WO-A2-2010/040023
- US-A- 843 505
- US-A- 61/7 33, 879
- US-A1- 2003 104 479
- US-A1- 2006 198 820
- US-A1- 2009 269 368
- US-A1- 2010 099 613
- US-A1- 2010 330 121
- US-A1- 2012 213 811
- PETER HEGER ET AL: "Qualitative Highly Divergent Nuclear Export Signals Can Regulate Export by the Competition for Transport Cofactors in Vivo", TRAFFIC, vol. 2, no. 8, 1 August 2001 (2001-08-01), pages 544-555, XP055276447, DK ISSN: 1398-9219, DOI: 10.1034/j.1600-0854.2001.20804.x
- LACOUR ET AL.: 'Analysis and prediction of leucine-rich nuclear export signals' PROTEIR ENGINEERING, DESIGN & SELECTION vol. 17, no. 6, 2004, pages 527 - 536, XP055256199
- ZHANG ET AL.: 'HER2-targeting recombinant protein with truncated pseudomonas exotoxin A translocation domain efficiently kills breast cancer cells' CANCER BIOLOGY & THERAPY vol. 7, no. 8, August 2008, pages 1226 - 1231 ;, XP055094470
- SHIN J ET AL.: "Signals for retention of transmembrane proteins in the endoplasmatic reticulum studies with CD4 truncation mutants", PROC. NATL. ACAD. SCI, vol. 88, March 1991 (1991-03), pages 1918-1922,
- MEDINA-GODOY S. ET AL.: "Endoplasmatic reticulum-retention C-terminal sequence enhancs prodution of an 11S seed globulin from Amaranthus hypochondriacus in Pichia pastoris", BIOTECH. J., vol. 1, no. 10, 2006, pages 1085-1092,

## Description

### FIELD OF THE INVENTION

The present invention relates generally to fusion proteins and immunology.

### BACKGROUND OF THE INVENTION

Molecular biology has enabled the production of subunit vaccines, in which the immunogen is a fragment or a subunit of a parent protein or complex. The development of a stable vaccine that could elicit T cell sensitizing responses, and be flexible enough to incorporate sequences from many strains of an infectious agent would be desirable.

US patent publication No. 20120213811 A1 discloses a fusion protein comprising: a) a *Pseudomonas Exotoxin* A (PE) peptide comprising a binding domain and a PE translocation domain, the PE peptide being devoid of cytotoxic domain III; b) optionally gag24, being fused to the PE peptide; c) a fragment of gp120 C1 domain, being fused to the PE peptide or fused to the gag24 if the gag24 is present; d) a fragment of gp 120 C5 domain, being fused to the fragment of gp120 C1 domain; e) a fragment of gp41 amino acid sequence, being fused to the fragment of gp 120 C5 domain, and f) optionally an endoplasmic reticulum retention sequence, being fused to the C-terminus of the fragment of gp41.

LaCour et al. published results of analysis and prediction of leucine-rich nuclear export signals (Protein Engineering, Design & Selection vol. 17 no. 6 pp. 527-536, 2004).

US patent publication No. 2010/0099613 A1 discloses immunogenic fusion proteins and protein comprising a nuclear export signal comprising the amino acid sequence of SEQ ID NO: 13 (LXXKLXXLXL).

Zhang et al. published HER2-targeting recombinant protein with truncated pseudomonas exotoxin A translocation domain efficiently kills breast cancer cells (Cancer Biology & Therapy 7:8, 1226-1231; August 2008).

Takemoto et al (J. Controlled Release, 2009, 135:11-18) disclosed enhanced generaton of cytotoxic T lymphocytes by increased cytosolic delivery of MHC class I epitope fused to mouse heat shock protein 70 via polyhistidine conjugation.

Manoharan et al (US 2005/0119470) disclosed Conjugated oligomeric compounds and their use in gene modulation.

Fukuda et al (J. Biol. Chem. 1996, 271 (33) 20024-20028) disclosed cytoplasmic localization of mitogen-activated protein kinase kinase directed by its NH2-terminal, leucine-rich short amino acid sequence, which acts as a nuclear transport signal.

WO 2010/040023 A2 discloses methods of protein therapy and treating diseases using a TUS protein, a NLS or NES identified from full length TUS.

WO 00/61772 A2 discloses methods for generating immune responses utilizing alphavirus-based vector systems.

Peter Heger et al (Traffic, vol. 2, no. 8, pages 544-555) discloses qualitative highly divergent nuclear export signals can regulate export by the composition for transport cofactors *in vivo.*

### SUMMARY OF THE INVENTION

In one aspect, the invention relates to a fusion protein comprising:
(a) an antigen-presenting cell (APC)-binding domain or a CD91 receptor-binding domain consisting of an amino acid sequence that is at least 90% identical to the sequence selected from the group consisting of SEQ ID NOs: 1 and 8-11, located at the N-terminus of the fusion protein;
(b) a translocation peptide of 34-112 amino acid residues in length, consisting of an amino acid sequence that is at least 90% identical to SEQ ID NO: 4, 2, 3, or 6, located at the C-terminus of the APC-binding domain or the CD91 receptor-binding domain; and
(c) an antigen of a pathogen;
(d) a nuclear export signal, comprising the amino acid sequence of SEQ ID NO: 13, wherein the C-terminal amino acid of the SEQ ID NO: 13 is alanine; and
(e) an endoplasmic reticulum retention sequence of four amino acid residues in length, located at the C-terminus of the fusion protein; wherein the nuclear export signal is located between the antigen and the endoplasmic reticulum retention sequence, or between the translocation peptide and the antigen.

In one embodiment of the invention, the APC-binding domain or the CD91 receptor-bindingdomain consists of the amino acid sequence of SEQ ID NO: 1, 8, 9, 10, or 11.

In another embodiment of the invention, the nuclear export signal comprises the amino acid sequence of SEQ ID NO: 14.

In another embodiment of the invention, the endoplasmic reticulum retention sequence consists of the amino acid sequence of SEQ ID NO: 15.

In another embodiment of the invention, the nuclear export signal is located between the translocation peptide and the antigen.

In another embodiment of the invention, the nuclear export signal is located between the antigen and the endoplasmic reticulum retention sequence.

In another embodiment of the invention, the nuclear export signal and the ER retention sequence forms a fusion peptide consisting of an amino acid sequence of SEQ ID NO: 12.

In another embodiment of the invention, the translocation peptide has 34-61 amino acid residues in length.

Also disclosed is a case in which the translocation peptide has 34-46 amino acid residues in length.

In another embodiment of the invention, the APC-binding domain or the CD91 receptor-binding domain is free of the amino acid sequence of Pseudomonas exotoxin A (PE) binding domain I.

In another embodiment of the invention, the APC-binding domain or the CD91 receptor-binding domain consists of the amino acid sequence of SEQ ID NO: 8.

In another embodiment of the invention, the amino acid sequence of the APC-binding domain or the CD91 receptor-binding domain is SEQ ID NO: 1 and the amino acid sequence of the translocation peptide is at least 90% identical to SEQ ID NO: 4, 2, or 3.

In another embodiment of the invention, the antigen is a fusion antigen of two or more antigenic peptides from a pathogen.

In one aspect of the disclosure, the translocation peptide consists of an amino acid sequence that is at least 95% identical to SEQ ID NO: 4, 2, 3, or 6.

In a specific embodiment of the invention, the translocation peptide is of 34-61 amino acid residues in length and consists of an amino acid sequence that is at least identical to SEQ ID NO: 4, 2, or 3.

In further aspects of the disclosure, the APC-binding domain or the CD91 receptor-binding domain exhibits a characteristics of recognizing and binding to a receptor on an antigen-presenting cell (APC) selected from the group consisting of dendritic cells, monocytes, B-cells and lymphocytes.

In further aspects of the disclosure, the pathogen is selected from the group consisting of PRRSV, PCV, FMDV, CSFV, NDV, Transmissible gastroenteritis virus (TGEV), Porcine epidemic diarrhea virus (PEDV), Influenza virus, Pseudorabies virus, Prvovirus, Pseudorabies virus, Swine vesicular disease virus (SVDV), Poxvirus, Rotavirus, Mycoplasma pneumonia, Herpes virus, Infectious bronchitis, and Infectious bursal disease virus.

Further in another aspect, the invention relates to a vaccine composition comprising the fusion protein as aforementioned and an adjuvant.

Yet in another aspect, the disclosure provides a method for inducing enhanced pathogen antigen-specific T cell responses, comprising: administering a vaccine composition comprising a therapeutically effective amount of the fusion protein as aforementioned to a subject in need thereof, and thereby inducing enhanced pathogen antigen-specific T cell responses.

The invention also relates to a fusion protein or a vaccine composition as aforementioned for use in the treatment of infections by inducing enhanced pathogen antigen-specific T cell responses.

### BRIEF DESCRIPTION OF THE DRAWINGS

FIG. 1A is a schematic drawing showing a full-length Pseudomonas aeruginosa exotoxin A (PE), and partial fragment of PE.
FIGs. 1B-C show vector maps.
FIGs. 2-5 are graphs showing fusion proteins according to the invention eliciting enhanced CD8⁺/ IFN-γ ⁺ T cell (FIGs. 2A-5A) and CD4⁺ / IFN-γ ⁺ T cell (FIGs. 2B-5B) mediated immunogenicities, respectively.
FIG. 6 shows animal groups, vaccines and dosage used for immunizing the animals, and immunization schedules.
FIGs. 7-8 are graphs showing tumor size curves and percentage of tumor-free mice in the animal groups vaccinated with various fusion proteins or placebo, respectively.

### DETAILED DESCRIPTION OF THE INVENTION

The present invention is more particularly described in the following examples that are intended as illustrative only since numerous modifications and variations therein will be apparent to those skilled in the art. Various embodiments of the invention are now described in detail. Referring to the drawings, like numbers indicate like components throughout the views. As used in the description herein and throughout the claims that follow, the meaning of "a", "an", and "the" includes plural reference unless the context clearly dictates otherwise. Also, as used in the description herein and throughout the claims that follow, the meaning of "in" includes "in" and "on" unless the context clearly dictates otherwise. Moreover, titles or subtitles may be used in the specification for the convenience of a reader, which shall have no influence on the scope of the present invention. Additionally, some terms used in this specification are more specifically defined below.

### DEFINITIONS

The terms used in this specification generally have their ordinary meanings in the art, within the context of the invention, and in the specific context where each term is used. Certain terms that are used to describe the invention are discussed below, or elsewhere in the specification, to provide additional guidance to the practitioner regarding the description of the invention. For convenience, certain terms may be highlighted, for example using italics and/or quotation marks. The use of highlighting has no influence on the scope and meaning of a term; the scope and meaning of a term is the same, in the same context, whether or not it is highlighted. It will be appreciated that same thing can be said in more than one way. Consequently, alternative language and synonyms may be used for any one or more of the terms discussed herein, nor is any special significance to be placed upon whether or not a term is elaborated or discussed herein. Synonyms for certain terms are provided. A recital of one or more synonyms does not exclude the use of other synonyms. The use of examples anywhere in this specification including examples of any terms discussed herein is illustrative only.

Unless otherwise defined, all technical and scientific terms used herein have the same meaning as commonly understood by one of ordinary skill in the art to which this invention pertains. In the case of conflict, the present document, including definitions will control.

The term "an antigen-presenting cell (APC) or accessory cell" refers to a cell that displays foreign antigens complexed with major histocompatibility complexes (MHC's) on their surfaces. T-cells may recognize these complexes using their T-cell receptors (TCRs). These cells process antigens and present them to T-cells. Main types of professional antigen-presenting cell are dendritic cells (DCs), macrophages, which are also CD4+ and are therefore also susceptible to infection by HIV; monocytes, and certain B-cells.

The term "an antigen-presenting cell (APC)-binding domain" refers to a domain (which is a polypeptide) that can bind to an antigen-presenting cell (APC). The APC-binding domain may be a polypeptide comprising an amino acid sequence that is at least 90% identical to the sequence selected from the group consisting of SEQ ID NOs: 1 and 8-11. An APC-binding domain is a ligand that recognizes and binds to a receptor on APC.

Cluster of differentiation 91 (CD91) is a protein that forms a receptor in the membrane of cells and is involved in receptor-mediated endocytosis.

The term "PEₜ" refers to a translocation peptide (or a translocation domain) with 34-112 amino acid residues in length. PEₜ may comprises the amino acid sequence that is at least. 90% identical to SEQ ID NO: 2-4 and 6. For example, the amino acid sequence of PEₜ may be a fragment of a. a 280 - a.a. 313 (SEQ ID NO: 4), a.a. 268 - a.a. 313 (SEQ ID NO: 3), a.a. 253 - a.a. 313 (SEQ ID NO: 2), or a.a. 253 - a.a. 364 (SEQ ID NO: 6) of PE. That is, the amino acid sequence of PEₜ may contain any region of the PE domain II (a.a. 253 to a.a. 364: SEQ ID NO: 6) as long as it comprises a. a 280-a.a. 313 (SEQ ID NO: 4) essential sequence (i.e., the essential fragment).

The PE₄₀₇ (SEQ ID NO. 7) is described in prior patent (US 7,335,361 B2) as PE(ΔIII).

The term "minimum translocation peptide" refers to PE₂₅₃₋₃₁₃ (SEQ ID NO. 2), which can translocate an antigen into the cytoplasm of a target cell.

The term "an endoplasmic reticulum (ER) retention sequence" refers to a peptide whose function is to assist translocation of an antigen from the cytoplasm into ER and retains the antigen in the lumen of the ER. An ER retention sequence comprises the sequence of Lys Asp Glu Leu (KDEL; SEQ ID NO: 15) or RDEL, An ER retention sequence may comprise the sequence KDEL, RDEL, KDELKDELKDEL (K3; SEQ ID NO: 16), KKDLRDELKDEL (K3; SEQ ID NO: 17), KKDELRDELKDEL (K3; SEQ ID NO: 18), or KKDELRVELKDEL (K3; SEQ ID NO: 19).

A nuclear export signal (NES) refers to a short amino acid sequence of 4 hydrophobic residues in a protein that targets it for export from the cell nucleus to the cytoplasm through the nuclear pore complex using nuclear transport. The NES is recognized and bound by exporting. The most common spacing of the hydrophobic residues to be LₓₓKLₓₓLₓLₓ (SEQ ID NO. 13), where "L" is leucine, "K" is lysine and "x" is any naturally occurring amino acid. For example, an artificial NES may comprise the sequence Leu Gln Lys Lys Leu Glu Glu Leu Glu Leu Ala (LQKKLEELELA; SEQ ID NO: 14).

The term "NESK" refers to a fusion peptide of a NES and an ER retention signal (i.e., a NES fused to an ER retention signal). It is an artificial peptide possessing the function of a nuclear export signal (NES) and an ER retention sequence. Thus, it can export an antigen from the cell nucleus to the cytoplasm through the nuclear pore complex, and assist translocation of an antigen front the cytoplasm to ER and retain the antigen in the lumen of the ER. For example, the amino acid sequence of NESK may be LQKKLEELELAKDEL (SEQ ID NO: 12).

An antigen may be a pathogenic protein, polypeptide or peptide that is responsible for a disease caused by the pathogen, or is capable of inducing an immunological response in a host infected by the pathogen, or tumor-associated antigen (TAA) which is a polypeptide specifically expressed in tumor cells. The antigen may be selected from a pathogen or cancer cells including, but not limited to, Human Papillomavirus (HPV), Porcine reproductive and respiratory syndrome virus (PRRSV), Human immunodeficiency virus-1(HIV-1), Dangue virus, Hepatitis C virus (HCV), Hepatitis B virus (HBV), Porcine Circovirus 2 (PCV2), Classical Swine Fever Virus (CSFV), Foot-and-mouth disease virus (FMDV), Newcastle disease virus (NDV), Transmissible gastroenteritis virus (TGEV), Porcine epidemic diarrhea virus (PEDV), Influenza virus. Pseudorabies virus, Prvovirus, Pseudorabies virus, Swine vesicular disease virus (SVDV), Poxvirus, Rotavirus, Mycoplasma pneumonia, Herpes virus, infectious bronchitis, or infectious bursal disease virus, non-small cell lung cancer, breast carcinoma, melanoma, lymphomas, colon carcinoma, hepatocellular carcinoma and any combination thereof. For example, HPV E7 protein (E7), HCV core protein (HCV core), HBV X protein (HBx) were selected as antigens for vaccine development. The antigen may be a fusion antigen from a fusion of two or more antigens selected from one or more pathogenic proteins. For example, a fusion antigen of PRRSV ORF6 and ORF5 fragments, or a fusion of antigenic proteins from PRRSV and PCV2 pathogens.

The term "treating" or "treatment" refers to administration of an effective amount of the fusion protein to a subject in need thereof, who has cancer or infection, or a symptom or predisposition toward such a disease, with the purpose of cure, alleviate, relieve, remedy, ameliorate, or prevent the disease, the symptoms of it, or the predisposition towards it. Such a subject can be identified by a health care professional based on results from any suitable diagnostic method.

The term "an effective amount" refers to the amount of an active compound that is required to confer a therapeutic effect on the treated subject. Effective doses will vary, as recognized by those skilled in the art, depending on rout of administration, excipient usage, and the possibility of co-usage with other therapeutic treatment.

The invention relates to fusion proteins for enhancing antigen delivery and modulating cell-mediated immune response. The fusion protein comprises: (a) an antigen-presenting cell (APC)-binding domain or a CD91 receptor-binding domain, located at the N-termimis of the fusion protein; (b) a translocation peptide of 34-112 amino acid residues in length, comprising an amino acid sequence that is at least 90% identical to SEQ ID NO: 2-4 and 6 and located at the C-terminus of the APC-binding domain or the CD91 receptor-binding domain; and (c) an antigen of a pathogen, located at the C-terminus of the translocation peptide; (d) a nuclear export signal (NES); and (e) an endoplasmic reticulum (ER) retention sequence, the ER retention sequence being located at the C-terminus of the fusion protein, wherein the NES comprises the amino acid sequence of SEQ ID NO: 13.

Using the fusion protein PE₃₁₃-ORF2-NESK as an example, the strategy is that the fusion protein of the invention stimulates the production and activation of T cells that can recognize the antigen Porcine Circovirus Type 2 (PCV2) capsid protein ORF2. The fusion protein comprises, from N-terminus to C-terminus, a PE domain I (APC-binding domain), a translocation peptide of 34-112 amino acid residues in length (e.g., a.a. 253-313 of the PE domain II), a truncated PCV2 ORF2 protein (N-terminal nuclear localization signal removed), a NES signal and an ER retention sequence (KDEL). The underlying mechanisms of eliciting enhanced ORF2-specific T cell immune responses by PE₃₁₃-ORF2-NESK involve the following steps: a) binding to dendritic cell (or antigen-presenting cell) surface receptor (CD91); b) internalization by endocytosis; c) transporting to the ER and proteolytic hydrolysis by furin in front of the translocation peptide; d) processing and presenting by MHC I complex; and e) activating antigen-specific CD4+ and CD8+ T cells. CD4+ Th1 cells are able to efficiently stimulate and enhance cytotoxic CD8+ T cell immune response. Together, these two arms of the adaptive immune system have the specificity and potency to kill PCV2 and PCV2-infected cells.

The fusion protein PE₃₁₃-ORF2-NESK here is distinguishable from the fusion protein vaccine PE₄₀₇-Ag-K3 disclosed by Lai in US 7,335,361 in several aspects. Firstly, the length of PE₃₁₃ (SEQ ID NO: 5) is 94 amino acid residues shorter than PE₄₀₇ (SEQ ID NO: 7), the advantage of which is that unwanted humoral response elicited by the presence of an extra fragment of PE is minimized or eliminated. Secondly, the ER retention sequence is shortened. Instead of K3 (that is, 3 of KDER), only one KDER or RDER is needed. Thirdly, only cytosolic antigen can be processed and presented by MHC type I pathway, so the addition of a NES signal into the fusion protein is beneficial to enhance pathogen antigen-specific T cell responses because increasing the opportunity of translocation of antigen into cytosol. Antigens of a pathogen may be imported into the cell nucleus. By incorporating a NES signal, the antigen imported into the cell nucleus can be exported to the cytoplasm by the NES signal of the fusion protein.

### EXAMPLES

Exemplary instruments, apparatus, methods and their related results according to the embodiments of the present invention are given below.

### EXAMPLE 1

### Construction of Expression Vectors

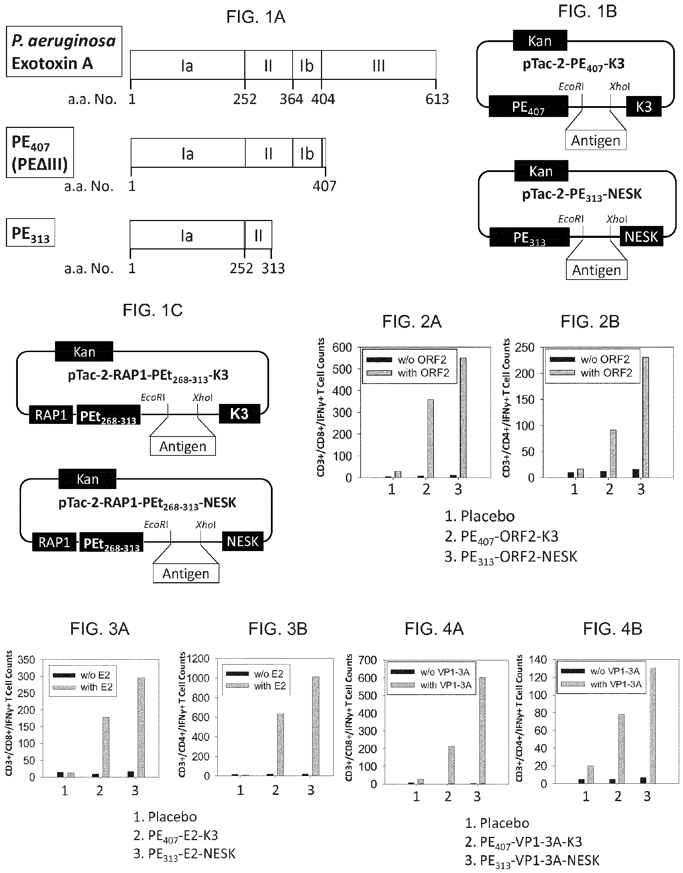

FIG. 1.A shows PE contains 3 domains (I, II, and III). PE₄₀₇ is the region from a.a. 1 to a.a 407 of PE. PE₄₀₇ does not contain the cytotoxic domain III and thus contains domains I and II. PE₃₁₃ is the region from a.a. 1 to a.a. 313 of PE. Thus, PE₃₁₃ contains only domain Ia and a partial N-termmal region of domain II of PE.

FIGs. 1B-C show constructions of expression vectors, each of which comprises an antigen-presenting cell (APC)-binding domain, a translocation peptide, an antigen, with (bottom panel) or without (top panel) a nuclear export signal (NES); and an endoplasmic reticulum (ER) retention sequence (top panel, K3 or bottom panel, K), the ER retention sequence being located at the C-terminus of the fusion protein. The plasmids pTac-2-PE₃₁₃-NESK, pTac-2-PE₄₀₇-K3, pTac-2-RAP1-PE₂₆₈₋₃₁₃-NESK and pTac-2-RAP1-PE₂₆₈₋₃₁₃-K3 were generated as follows: The ^{NdeI}PE₃₁₃-^{(EcoRI,XhoI)}-NESK^{XhoI}, ^{NdeI}PE₄₀₇-^{(EcoRI,XhoI)}-K3^{XhoI}, ^{NedI}RAP1-^{(EcoRI)}-PE₂₆₈₋₃₁₃-^{(EcoRI,XhoI)}-NESK^{XhoI} and ^{NdeI}RAP1-^{(EcoRI)}-PE₂₆₈₋₃₁₃-^{(EcoRI,XhoI)}-K3^{XhoI} fragments were synthesized by a PCR method and then ligated into a pUC18 back bond with kanamycin resistance gene to obtain respective plasmids.

A target DNA encoding an antigen or a fusion antigen of a pathogen of interest may then be inserted into the aforementioned plasmids it generate an expression vector for expression of a fusion protein. For example, DNA fragments encoding antigens of Porcine Circovirus Type 2 (PCV2) ORF2 (SEQ ID NO: 20), Classical Swine Fever Virus (CSFV) E2 (SEQ ID NO: 21), Foot-and-mouth disease virus (FMDV) VP1-3A (SEQ ID NO: 24) and Newcastle disease virus (NDV) FHN (SEQ ID NO: 27) were synthesized and inserted into the plasmids pTac-2-PE₃₁₃-NESK and pTac-2-PE₄₀₇-K3, respectively, to generate the following expression vectors: (1) PE₃₁₃-ORF2-NESK;(2) PE₄₀₇-ORF2-K3;(3)PE₃₁₃-E2-NESK;(4)PE₄₀₇-E2-K3;(5) PE₃₁₃-VP1-3A-NESK;(6) PE₄₀₇-VP1-3A-K3;(7) PE₃₁₃-FHN-NESK; and (8) PE₄₀₇-FHN-K3. DNA fragments encoding antigen of Human Papillomavirus Type 16 E7 (SEQ ID NO: 28) were synthesized and inserted into the plasmids pTac-2-PE₄₀₇-K3, pTac-2-RAP1-PE₂₆₈₋₃₁₃-NESK and pTac-2-RAP1-PE₂₆₈₋₃₁₃-K3, respectively, to generate the following expression vectors: (9) PE₄₀₇-E7-K3, (10) RAP1-PE₂₆₈₋₃₁₃-E7-NESK and (11) RAP1-PE₂₆₈₋₃₁₃-E7-K3.

### EXAMPLE 2

### Protein expression

*E*. *coli* BL21 cells harboring plasmids for expression of fusion proteins (1) PE₃₁₃-ORF2-NESK; (2) PE₄₀₇-ORF2-K3; (3) PE₃₁₃-E2-NESK; (4) PE₄₀₇-E2-K3; (5) PE₃₁₃-VP1-3A-NESK; (6) PE₄₀₇-VP1-3A-K3; (7) PE₃₁₃-FHN-NESK; (8) PE₄₀₇-FHN-K3; (9) PE₄₀₇-E7-K3; (10) RAP1-PE₂₆₈₋₃₁₃-E7-NESK and (11) RAP1-PE₂₆₈₋₃₁₃-E7-K3 were respectively cultured in Luria Bertani broth containing 25 ppm of kanamycin at 37° C. When the culture reaching early log phase, (A600=0.1 to 0.4), isopropyl-1-thio-β-D-galactopyranoside (IPTG) was added with a final concentration of 0.5 to 2 mM for induction. Cells were harvested after induction after 4 hours and immediately stored at -70° C. The fusion proteins were purified by urea extraction as described previously (Liao et al., 1995. Appl, Microbiol. Biotechnol. 43: 498-507) and then were refolded by dialysis method against 50X volume of TNE buffer (50mM: Tris, 50mM NaCl and 1mM EDTA) at 4°C for overnight. The refolded proteins were subjected to SDS-PAGE analyses and quantitative analyses performed using Bradford Protein Assay Kit (Pierce). The results indicated that most of the refolded proteins were monomers under a non-reduced condition, indicating that the fusion proteins refolded easily and were not aggregated.

### EXAMPLE 3

### PCV2 subunit vaccines immunogenicity assay

Mice were vaccinated with 0.1 ml PCV2 subunit vaccine containing 40µg of PE₃₁₃-ORF2-NESK or PE₄₀₇-ORF2-K3 with aluminum phosphate (a protein absorbent for slow release of the fusion protein; 10% v/v) and 10µg of saponin (an adjuvant extracted from Quillaja saponaria) via s.c. injection once a week for 3 weeks. The control group (placebo) was injected with adjuvant only without the fusion protein. All mice were sacrificed 14 days after the last immunization, and the spleens were harvested. The splenocytes were isolated and cultured in 6-well plate (10⁸ cells/2ml/well) with or without the recombinant ORF2 protein in the presence of 1 µg/ml GolgiPlug (BD Pharmingen, San Diego, CA) at 37°C for 16 hr. The stimulated splenocytes were then washed with FACScan buffer and stained with phycoerythrin-conjugated monoclonal rat anti-mouse CD8a and AF700-conjugated monoclonal rat anti-mouse CD4 antibodies. Cells were intracellular cytokine stained using the Cytofix/Cytoperm kit according to the manufacturer's instructions (BD Pharmingen). Intracellular IFN-γ was stained with AF488-conjugated rat anti-mouse IFN-γ to measure the immune response and cytokine levels. Flow cytometry analyses were performed using Gallios flow cytometry with Kaluza analysis software (Beckman Coulter).

FIGs. 2A-B show the numbers of CD8 and CD4 positive IFN-γ T cells in the splenocytes from mice vaccinated with a placebo (adjuvant only without the fusion protein) or fusion proteins, respectively. The IFN-γ production by CD4+ and CD8+ T cells in splenocytes stimulated with ORF2 was detected by intracelluar staining via flow cytometry. Bar graphs show the numbers of ORF2-specific IFN-γ+ CD4+ T cells (FIG. 2B) and IFN-γ+ CD8+ T cells (FIG. 2A) from each group with (grey bars) or without (black bars) stimulation by the ORF2 peptide. The results indicated that the mice that had been vaccinated with PE₃₁₃-ORF2-NESK had more ORF2-specific CD4+ IFN-γ+ and CD8+ IFN-γ+ T cells stimulated by the ORF2 peptide than the mice that had been vaccinated with PE₄₀₇-ORF2-K3 group.

### EXAMPLE 4

### CSFV subunit vaccines immunogenicity assay

Using the same immunization schedule and dosage, mice were vaccinated with CSFV subunit vaccines containing PE₃₁₃-E2-NESK or PE₄₀₇-E2-K3, and splenocytes isolated, cultured and assayed by a flow cytometry method as described above, except that the recombinant E2 protein was added to stimulate the splenocytes in the culture.

FIGs. 3A-B show the numbers of CD8 and CD4 positive IFN-γ T cells in the splenocytes from mice vaccinated with a placebo (adjuvant only without the fusion protein) or fusion proteins, respectively. The IFN-γ production by CD4+ and CD8+ T cells in splenocytes stimulated with B2 was detected by intracelluar staining via flow cytometry. Bar graphs show the numbers of E2-specific IFN-γ+ CD4+ T cells (FIG. 3B) and IFN-γ+ CD8+ T cells (FIG. 3A) from each group with (grey bars) or without (black bars) stimulation by the E2 peptide. The results indicated that the mice that had been vaccinated with PE₃₁₃-E2-NESK had more E2-specific CD4+ IFN-γ+ and CD8+ IFN-γ+ T cells stimulated by the E2 peptide than the mice that had been vaccinated with PE₄₀₇-E2-K3 group.

### EXAMPLE 5

### FMDV subunit vaccines immunogenicity assay

Using the same immunization schedule and dosage, mice were vaccinated with FMDV subunit vaccines containing PE₃₁₃ VP1-3A-NESK or PE₄₀₇- VP1-3A-K3, and splenocytes isolated, cultured and assayed by a flow cytometry method as described above, except that the recombinant VP1-3A protein was added to stimulate the splenocytes in the culture.

FIGs. 4A-B show the numbers of CD8 and CD4 positive IFN-γ T cells in the splenocytes from mice vaccinated with a placebo or fusion proteins. The IFN-γ production by CD4+ and CD8+ T cells in splenocytes stimulated with VP1-3A was detected by intracelluar staining via flow cytometry. Bar graphs show the numbers of VP1-3A-specific IFN-γ+ CD4+ T cells (FIG. 4B) and IFN-γ+ CD8+ T cells (FIG. 4A) from each group with (grey bars) or without (black bars) stimulation by the VP1-3A peptide. The results indicated that the mice that had been vaccinated with PE₃₁₃- VP1-3A-NESK had more VP1-3A-specific CD4+ IFN-γ+ and CD8+ IFN-γ+ T cells stimulated by the VP1-3A peptide than the mice that had been vaccinated with PE₄₀₇- VP1-3A-K3 group.

### EXAMPLE 6

### NDV subunit vaccines immunogenicity assay

Using the same immunization schedule and dosage, mice were vaccinated with FMDV subunit vaccines containing PE₃₁₃- FHN-NESK or PE₄₀₇-FHN-K3, and splenocytes isolated, cultured and assayed by a flow cytometry method as described above, except that the recombinant FHN protein was added to stimulate the splenocytes in the culture.

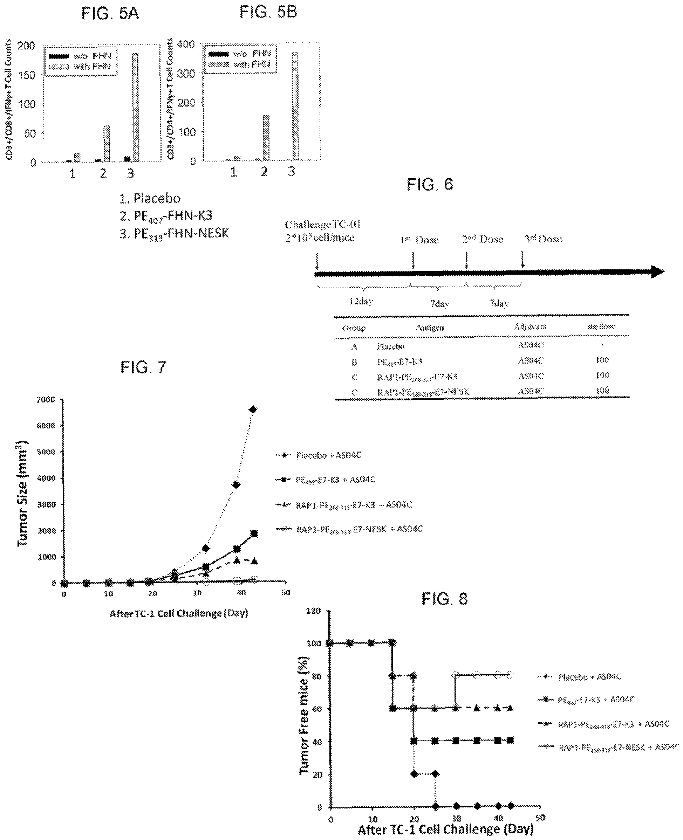

FIGs. 5A-B show the numbers of CD8 and CD4 positive IFN-γ T cells in the splenocytes from mice vaccinated with a placebo or fusion proteins. The IFN-γ production by CD4+ and CD8+ T cells in splenocytes stimulated with FHN was detected by intracelluar staining via flow cytometry. Bar graphs show the numbers of FHN-specific IFN-γ+ CD4+ T cells (FIG. 5B) and IFN-γ+ CD8+ T cells (FIG. 5A) from each group with (grey bars) or without (black bars) stimulation by the FUN peptide. The results indicated that the mice that had been vaccinated with PE₃₁₃- FHN-NESK had more FHN specific CD4+ IFN-γ+ and CD8+ IFN-γ+ T cells stimulated by the FHN peptide than the mice that had been vaccinated with PE₄₀₇-FHN-K3 group.

### EXAMPLE 7

Enhanced inhibition of tumor growth induced by human papilloma virus type 16 E7 protein The fusion proteins PE₄₀₇-E7-K3, RAP1-PE₂₆₈₋₃₁₃-E7-K3 and RAP1-PE₂₆₈₋₃₁₃-E7-NESK were expressed and refolded using similar methods as described above. Mice were challenged with 2 x 10³ TC-01 cells (mouse lung epithelia cell harboring HPV type 16 E7 gene) via s.c. injection to induce HPV-16 type carcinoma. Twelve days after the TC-01 cell challenge, mice were vaccinated via s.c. with placebo (PBS), PE₄₀₇-E7-K3 (100 mg/dose), RAP1-PE₂₆₈₋₃₁₃-E7-K3 (100 µg/dose) or RAP1-PE₂₆₈₋₃₁₃-E7-NESK (100 µg/dose) with AS04C (GlaxoSmithKline) as an adjuvant once per week for 3 weeks (FIG. 6). AS04C, which is a cytotoxic T lymphocyte-enhancing adjuvant, comprises MPL (monophosphoryl lipid A, an immune potentiator) and aluminum phosphate(a protein absorbent for antigen delivery). The term "K3" refers to an ER retention sequence comprising KDEL. For example, K3 may be the amino acid sequence KDELKDELKDEL (SEQ ID NO: 16). The term "NESK" refers to a fusion peptide comprising a nuclear export signal and an ER retention sequence. In one embodiment of the invention, the NESK is the amino acid sequence LQKKLEELELAKDEL (SEQ ID NO: 12). The size of tumors and the number of tumor-free animals in each group were recorded (FIGs. 7 and 8). The tumor growth was significantly suppressed by vaccines PE₄₀₇-E7-K3, RAP1-PE₂₆₈₋₃₁₃-E7-K3 and RAP1-PE₂₆₈₋₃₁₃-E7-NESK with AS04C as an adjuvant. However, the vaccine RAP1-PE₂₆₈₋₃₁₃-E7-NESK was superior to PE₄₀₇-E7-K3 and better than RAP1-PE₂₆₈₋₃₁₃-E7-K3 in suppressing tumor growth and increasing the percentage of tumor-free animals.

### EXAMPLE 8

The following fusion proteins are generated: PE₃₁₃-NES-antigen-K, PE₁₋₂₅₂-PE₂₆₈₋₃₁₃-NES-antigen-K, PE₁₋₂₅₂-PE₂₈₀₋₃₁₃-NES-antigen-K. In addition, the fragment of PE domain Ia (PE₁₋₂₅₂) of the fusion protein PE₃₁₃-antigen-NESK is replaced by RAP1 domain 3 (SEQ ID NO: 8), A2M minimum (SEQ ID NO: 9), HIV-Tat minimum (SEQ ID NO: 10) or HSPs minimum (SEQ ID NO: 11) to generate the fusion proteins RAP1 domain 3-PE₂₅₃₋₃₁₃-antigen-NESK, A2M-PE₂₅₃₋₃₁₃-antigen-NESK, Tat- PE₂₅₃₋₃₁₃-antigen -NESK and HSP- PE₂₅₃₋₃₁₃-antigen-NESK, RAP1 domain 3-PE₂₆₈₋₃₁₃-antigen-NESK, A2M-PE₂₆₈₋₃₁₃-antigen-NESK, Tat- PE₂₆₈₋₃₁₃-antigen -NESK and HSP-PE₂₆₈₋₃₁₃-antigen-NESK vaccines, RAP1 domain S-PE₂₈₀₋₃₁₃-antigen-NESK, A2M-PE₂₈₀₋₃₁₃-antigen-NESK, Tat- PE₂₈₀₋₃₁₃-antigen -NESK and HSP- PE₂₈₀₋₃₁₃-antigen-NESK, respectively. RAP1 domain 3-PE₂₅₃₋₃₁₃-NES-antigen-K, A2M-PE₂₅₃₋₃₁₃-NES-antigen-K, Tat- PE₂₅₃₋₃₁₃-NES-antigen -K and HSP-PE₂₅₃₋₃₁₃-NES-antigen-K, RAP1 domain 3-PE₂₆₈₋₃₁₃-NES-antigen-K, A2M-PE₂₆₈₋₃₁₃-NES-antigen-K, Tat- PE₂₆₈₋₃₁₃-NES-antigen -K and HSP- PE₂₆₈₋₃₁₃-NES-antigen-K vaccines, RAP1 domain 3-PE₂₈₀₋₃₁₃-NES-antigen-K A2M-PE₂₈₀₋₃₁₃-NES-antigen-K, Tat- PE₂₈₀₋₃₁₃-NES-antigen -K and HSP- PE₂₈₀₋₃₁₃-NES-antigen-K. The cell mediated immune responses enhanced by these vaccines are examined using similar methods as described above.

Table 1 shows SEQ ID NOs. of peptides used for making various fusion proteins.

**Table 1**

| Component | SEQ ID NO: | amino acid residues |
|---|---|---|
| Minimum Pseudomonas exotoxin A (PE) binding domain la (APC-binding domain, a.a.1- a.a.252 of PE) | 1 | 252 |
| PE₂₅₃₋₃₁₃ | 2 | 61 |
| PE₂₆₈₋₃₁₃ (translocation domain) | 3 | 46 |
| PEₜ Core (PE translocation domain core; a.a. 280- a.a. 313 of PE) | 4 | 34 |
| PE₃₁₃ (a.a. 1 - a.a. 313 of PE) | 5 | 313 |
| PE₂₅₃₋₃₆₄ | 6 | 112 |
| PE₄₀₇ (a.a. 1- a.a. 407 of PE) | 7 | 407 |
| RAP1 Minimum (domain III of RAP 1) | 8 | 104 |
| A2M Minimum | 9 | 153 |
| HIV-Tat Minimum | 10 | 24 |
| HSPs Minimum | 11 | 641 |
| NESK is **LQKKLEELELA**KDEL* | 12 | 15 |
| NES consensus sequence is L_{XX}KL_{XX}L_{X}L_{X}, wherein "L" is leucine, "K" is lysine and "x" is any naturally occurring amino acid | 13 | 11 |
| NES is LQKKLEELELA | 14 | 11 |
| KDEL | 15 | 4 |
| KDELKDELKDEL (K3) | 16 | 12 |
| KKDLRDELKDEL (K3) | 17 | 12 |
| KKDELRDELKDEL (K3) | 18 | 13 |
| KKDELRVELKDEL (K3) | 19 | 13 |
| PCV2 ORF2 (Porcine Circovirus type 2 Open Reading Frame 2) | 20 | 192 |
| CSFV E2 (Classical Swine Fever Virus Envelope glycoprotein E2) | 21 | 328 |
| FMDV VP1 peptide (viral capsid protein a.a. 127-a.a. 176 of VP1) | 22 | 50 |
| FMDV 3A peptide (a.a. 21-35 of 3A) | 23 | 15 |
| FMDV (Foot-and-Mouth Disease Virus) VP1-3A peptide** | 24 | 65 |
| NDV F peptide (a.a. 65- a.a. 82 of Fusion protein) | 25 | 18 |
| NDV HN peptide (a.a. 101- a.a. 111 of Hemagglutinin-Neuraminidase) | 26 | 11 |
| NDV FHN peptide *** | 27 | 29 |
| HPV (Human Papillomavirus) Type16 E7 | 28 | 98 |
| Full length PE (Exotoxin A, *Pseudomonas aeruginosa*) | 29 | 613 |

| | | |
|---|---|---|
| *: The hold letters represents the amino acid sequence of an artificial nuclear exporting signal: the underlined letters represents the amino acid sequence of an endoplasmic reticulum retention signal. **: The VP1-3A peptide is a fusion antigen composed of a.a. 127 - a.a. 176 of VP1 and a.a. 21 - a.a. 35 of 3A; i.e., a fusion of FMDV VP1 peptide (SEQ ID NO: 22) and FMDV 3A peptide (SEQ ID NO: 23). ***: The FHN peptide is a fusion antigen composed of a.a. 65 - a.a. 82 of fusion protein and (a.a. 101- a.a. 111 of Hemagglutinin-Neuraminidase; i.e., a fusion of NDV F peptide (SEQ ID NO: 25) and NDV HN peptide (SEQ ID NO: 26) | | |

In summary, the results have proved that a fusion protein containing an APC-binding domain at the N-terminal end, a translocation domain, followed by an antigen of a pathogen, and then a fusion peptide of NESK at the carboxyl terminal end is an improved design over the PE-fusion protein that is without the fusion peptide of NESK at the carboxyl terminus in terms of enhancing cell-mediated immune response, suppressing tumor growth, and/or increasing the percentage of tumor-free animals.

## Claims

1. A fusion protein comprising:
(a) an antigen-presenting cell (APC)-binding domain or a CD91 receptor-binding domain consisting of an amino acid sequence that is at least 90% identical to the sequence selected from the group consisting of SEQ ID NOs: 1 and 8-11, located at the N-terminus of the fusion protein;
(b) a translocation peptide of 34-112 amino acid residues in length, consisting of an amino acid sequence that is at least 90% identical to SEQ ID NO: 4, 2, 3, or 6, located at the C-terminus of the APC-binding domain or the CD91 receptor-binding domain; and
(c) an antigen of a pathogen;
(d) a nuclear export signal, comprising the amino acid sequence of SEQ ID NO: 13; and
(e) an endoplasmic reticulum retention sequence of four amino acid residues in length, located at the C-terminus of the fusion protein; wherein the nuclear export signal is located between the antigen and the endoplasmic reticulum retention sequence, or between the translocation peptide and the antigen.

2. The fusion protein of claim 1, wherein the APC-binding domain or CD91 receptor-binding domain consists of the amino acid sequence of SEQ ID NO: 1, 8, 9, 10, or 11.

3. The fusion protein of claim 1, wherein the nuclear export signal comprises the amino acid sequence of SEQ ID NO: 14.

4. The fusion protein of claim 1, wherein the endoplasmic reticulum retention sequence consists of the amino acid sequence of SEQ ID NO: 15.

5. The fusion protein of claim 1, wherein the nuclear export signal is located between the translocation peptide and the antigen.

6. The fusion protein of claim 1, wherein the nuclear export signal is located between the antigen and the endoplasmic reticulum retention sequence.

7. The fusion protein of claim 1, wherein the nuclear export signal and the ER retention sequence forms a fusion peptide consisting of an amino acid sequence of SEQ ID NO: 12.

8. The fusion protein of claim 1, wherein the translocation peptide has 34-61 amino acid residues in length.

9. The fusion protein of claim 1, wherein the APC-binding domain or the CD91 receptor-binding domain is free of the amino acid sequence of Pseudomonas exotoxin A (PE) binding domain I.

10. The fusion protein of claim 9, wherein the APC-binding domain or the CD91 receptor-binding domain consists of the amino acid sequence of SEQ ID NO: 8.

11. The fusion protein of claim 1, wherein the amino acid sequence of the APC-binding domain or the CD91 receptor-binding domain is SEQ ID NO: 1 and the amino acid sequence of the translocation peptide is at least 90% identical to SEQ ID NO: 4, 2, or 3.

12. The fusion protein of claim 1, wherein the antigen is a fusion antigen of two or more antigenic peptides from a pathogen.

13. The fusion protein of claim 1, wherein the translocation peptide is of 34-61 amino acid residues in length and consists of an amino acid sequence that is at least 90% identical to SEQ ID NO: 4, 2, or 3.

14. A vaccine composition comprising the fusion protein of claim 1 and an adjuvant.

15. A fusion protein as claimed in any one of claims 1-14 for use in the treatment of infections by inducing enhanced pathogen antigen-specific T cell responses.

## Patentansprüche

1. Fusionsprotein, umfassend:
(a) eine Bindungsdomäne einer antigenaufweisenden Zelle (antigen-presenting cell, APC) oder eine Bindungsdomäne des Rezeptors CD91 bestehend aus einer Aminosäuresequenz, die zu mindestens 90 % identisch ist mit der Sequenz, ausgewählt aus der Gruppe bestehend aus SEQ ID NO: 1 und 8-11, die sich am N-Terminus des Fusionsproteins befindet;
(b) ein Translokationspeptid mit einer Länge von 34-112 Aminosäureresten, bestehend aus einer Aminosäuresequenz, die zu mindestens 90 % identisch mit der SEQ ID NO: 4, 2, 3 oder 6 ist, die sich am C-Terminus der APC-Bindungsdomäne oder der Bindungsdomäne des Rezeptors CD91 befindet; und
(c) ein Antigen eines Pathogens;
(d) ein nukleäres Exportsignal, umfassend die Aminosäuresequenz von SEQ ID NO: 13; und
(e) eine endoplasmatische Retikulum-Retentionssequenz mit einer Länge von vier Aminosäureresten, die sich am C-Terminus des Fusionsproteins befindet; wobei das nukleäre Exportsignal zwischen dem Antigen und der endoplasmatischen Retikulum-Retentionssequenz oder zwischen dem Translokationspeptid und dem Antigen ist.

2. Fusionsprotein nach Anspruch 1, wobei die APC-Bindungsdomäne oder Bindungsdomäne des Rezeptors CD91 aus der Aminosäuresequenz von SEQ ID NO: 1, 8, 9, 10 oder 11 besteht.

3. Fusionsprotein nach Anspruch 1, wobei das nukleäre Exportsignal die Aminosäuresequenz von SEQ ID NO: 14 umfasst.

4. Fusionsprotein nach Anspruch 1, wobei die endoplasmatische Retikulum-Retentionssequenz aus der Aminosäuresequenz von SEQ ID NO: 15 besteht.

5. Fusionsprotein nach Anspruch 1, wobei sich das nukleäre Exportsignal zwischen dem Translokationspeptid und dem Antigen befindet.

6. Fusionsprotein nach Anspruch 1, wobei sich das nukleäre Exportsignal zwischen dem Antigen und der endoplasmatischen Retikulum-Retentionssequenz befindet.

7. Fusionsprotein nach Anspruch 1, wobei das nukleäre Exportsignal und die ER-Retentionssequenz ein Fusionspeptid bilden, das aus einer Aminosäuresequenz der SEQ ID

8. Fusionsprotein nach Anspruch 1, wobei das Translokationspeptid eine Länge von 34-61 Aminosäureresten aufweist.

9. Fusionsprotein nach Anspruch 1, wobei die APC-Bindungsdomäne oder die Bindungsdomäne des Rezeptors CD91 frei von der Aminosäuresequenz der Bindungsdomäne I von Pseudomonas-Exotoxin A (PE) ist.

10. Fusionsprotein nach Anspruch 9, wobei die APC-Bindungsdomäne oder die Bindungsdomäne des Rezeptors CD91 aus der Aminosäuresequenz von SEQ ID NO: 8 besteht.

11. Fusionsprotein nach Anspruch 1, wobei die Aminosäuresequenz der APC-Bindungsdomäne oder der Bindungsdomäne des Rezeptors CD91 SEQ ID NO: 1 ist, und die Aminosäuresequenz des Translokationspeptids zu mindestens 90 % identisch mit SEQ ID NO: 4, 2 oder 3 ist.

12. Fusionsprotein nach Anspruch 1, wobei das Antigen ein Fusionsantigen von zwei oder mehr antigenen Peptiden von einem Pathogen ist.

13. Fusionsprotein nach Anspruch 1, wobei das Translokationspeptid eine Länge von 34-61 Aminosäureresten aufweist und aus einer Aminosäuresequenz besteht, die zu mindestens 90 % identisch mit SEQ ID NO: 4, 2 oder 3 ist.

14. Impfstoffzusammensetzung, umfassend das Fusionsprotein nach Anspruch 1 und ein Adjuvans.

15. Fusionsprotein nach einem der Ansprüche 1-14 zur Verwendung in der Behandlung von Infektionen durch Induzieren von erhöhten pathogenantigen-spezifischen T-Zell-Reaktionen.

## Revendications

1. Protéine de fusion comprenant :
(a) un domaine de liaison aux cellules présentatrices d'antigène (CPA) ou un domaine de liaison au récepteur CD91 constitué par une séquence d'acides aminés qui est identique à au moins 90 % à la séquence sélectionnée dans le groupe constitué par les SEQ ID NO : 1 et 8 à 11, située à l'extrémité N-terminale de la protéine de fusion ;
(b) un peptide de translocation de 34 à 112 résidus d'acides aminés de longueur, constitué par une séquence d'acides aminés qui est identique à au moins 90 % à la SEQ ID NO : 4, 2, 3, ou 6, située à l'extrémité C-terminale du domaine de liaison aux CPA ou du domaine de liaison au récepteur CD91 ; et
(c) un antigène d'un pathogène ;
(d) un signal d'exportation nucléaire, comprenant la séquence d'acides aminés de la SEQ ID NO : 13 ; et
(e) une séquence de rétention au réticulum endoplasmique de quatre résidus d'acides aminés de longueur, située à l'extrémité C-terminale de la protéine de fusion ; dans laquelle le signal d'exportation nucléaire est situé entre l'antigène et la séquence de rétention au réticulum endoplasmique, ou entre le peptide de translocation et l'antigène.

2. Protéine de fusion selon la revendication 1, dans laquelle le domaine de liaison aux CPA ou le domaine de liaison au récepteur CD91 est constitué par la séquence d'acides aminés de la SEQ ID NO : 1, 8, 9, 10, ou 11.

3. Protéine de fusion selon la revendication 1, dans laquelle le signal d'exportation nucléaire comprend la séquence d'acides aminés de la SEQ ID NO : 14.

4. Protéine de fusion selon la revendication 1, dans laquelle la séquence de rétention au réticulum endoplasmique est constituée par la séquence d'acides aminés de la SEQ ID NO : 15.

5. Protéine de fusion selon la revendication 1, dans laquelle le signal d'exportation nucléaire est situé entre le peptide de translocation et l'antigène.

6. Protéine de fusion selon la revendication 1, dans laquelle le signal d'exportation nucléaire est situé entre l'antigène et la séquence de rétention au réticulum endoplasmique.

7. Protéine de fusion selon la revendication 1, dans laquelle le signal d'exportation nucléaire et la séquence de rétention au RE forment un peptide de fusion constitué par une séquence d'acides aminés de la SEQ ID NO : 12.

8. Protéine de fusion selon la revendication 1, dans laquelle le peptide de translocation fait 34 à 61 résidus d'acides aminés de longueur.

9. Protéine de fusion selon la revendication 1, dans laquelle le domaine de liaison aux CPA ou le domaine de liaison au récepteur CD91 est exempt de la séquence d'acides aminés du domaine de liaison I de l'exotoxine A de Pseudonomas (EP).

10. Protéine de fusion selon la revendication 9, dans laquelle le domaine de liaison aux CPA ou le domaine de liaison au récepteur CD91 est constitué par une séquence d'acides aminés de la SEQ ID NO : 8.

11. Protéine de fusion selon la revendication 1, dans laquelle la séquence d'acides aminés du domaine de liaison aux CPA ou du domaine de liaison au récepteur CD91 est la SEQ ID NO : 1 et la séquence d'acides aminés du peptide de translocation est identique à au moins 90 % à la SEQ ID NO : 4, 2, ou 3.

12. Protéine de fusion selon la revendication 1, dans laquelle l'antigène est un antigène de fusion de deux peptides antigéniques ou plus d'un pathogène.

13. Protéine de fusion selon la revendication 1, dans laquelle le peptide de translocation fait de 34 à 61 résidus d'acides aminés de longueur et est constitué d'une séquence d'acides aminés qui est identique à au moins 90 % à la SEQ ID NO : 4, 2, ou 3.

14. Composition de vaccin comprenant la protéine de fusion selon la revendication 1 et un adjuvant.

15. Protéine de fusion selon l'une quelconque des revendications 1 à 14 destinée à être utilisée dans le traitement d'infections en induisant des réponses améliorées de cellules T spécifiques d'un antigène de pathogène.


### SEQUENCE LISTING

<110> TheVax Genetics Vaccine Co., Ltd.
<120> FUSION PROTEINS FOR ENHANCING CELL-MEDIATED IMMUNE RESPONSES
<130> 10021-00003
<150> 61733879
   <151> 2012-12-05
<160> 29
<170> PatentIn version 3.5
<210> 1
   <211> 252
   <212> PRT
   <213> Pseudomonas aeruginosa
<400> 1 Met Gln Pro Thr Leu Ala Ile Ser His Ala Gly Val Ser Val Val Met
<210> 2
   <211> 61
   <212> PRT
   <213> Pseudomonas aeruginosa
<400> 2
   Gly Gly Ser Leu Ala Ala Leu Thr Ala His Gln Ala Cys His Leu Pro
   1 5 10 15
   Leu Glu Thr Phe Thr Arg His Arg Gln Pro Arg Gly Trp Glu Gln Leu
   20 25 30
   Glu Gln Cys Gly Tyr Pro Val Gln Arg Leu Val Ala Leu Tyr Leu Ala
   35 40 45
   Ala Arg Leu Ser Trp Asn Gln Val Asp Gln Val Ile Arg
   50 55 60
<210> 3
   <211> 46
   <212> PRT
   <213> Pseudomonas aeruginosa
<400> 3
   Pro Leu Glu Thr Phe Thr Arg His Arg Gln Pro Arg Gly Trp Glu Gln
   1               5                   10                  15
   Leu Glu Gln Cys Gly Tyr Pro Val Gln Arg Leu Val Ala Leu Tyr Leu
               20                  25                  30
   Ala Ala Arg Leu Ser Trp Asn Gln Val Asp Gln Val Ile Arg
           35                  40                  45
<210> 4
   <211> 34
   <212> PRT
   <213> Pseudomonas aeruginosa
<400> 4 Ile Arg
<210> 5
   <211> 313
   <212> PRT
   <213> Pseudomonas aeruginosa
<400> 5
   Ala Glu Glu Ala Phe Asp Leu Trp Asn Glu Cys Ala Lys Ala Cys Val
   1               5                   10                  15
   Leu Asp Leu Lys Asp Gly Val Arg Ser Ser Arg Met Ser Val Asp Pro
               20                  25                  30
   Ala Ile Ala Asp Thr Asn Gly Gln Gly Val Leu His Tyr Ser Met Val
           35                  40                  45
   Leu Glu Gly Gly Asn Asp Ala Leu Lys Leu Ala Ile Asp Asn Ala Leu
       50                  55                  60
   Ser Ile Thr Ser Asp Gly Leu Thr Ile Arg Leu Glu Gly Gly Val Glu
   65                  70                  75                  80
   Pro Asn Lys Pro Val Arg Tyr Ser Tyr Thr Arg Gln Ala Arg Gly Ser
                   85                  90                  95
   Trp Ser Leu Asn Trp Leu Val Pro Ile Gly His Glu Lys Pro Ser Asn
               100                 105                 110
   Ile Lys Val Phe Ile His Glu Leu Asn Ala Gly Asn Gln Leu Ser His
           115                 120                 125
   Met Ser Pro Ile Tyr Thr Ile Glu Met Gly Asp Glu Leu Leu Ala Lys
       130                 135                 140
   Leu Ala Arg Asp Ala Thr Phe Phe Val Arg Ala His Glu Ser Asn Glu
   145                 150                 155                 160
   Met Gln Pro Thr Leu Ala Ile Ser His Ala Gly Val Ser Val Val Met
                   165                 170                 175
   Ala Gln Thr Gln Pro Arg Arg Glu Lys Arg Trp Ser Glu Trp Ala Ser
               180                 185                 190
   Gly Lys Val Leu Cys Leu Leu Asp Pro Leu Asp Gly Val Tyr Asn Tyr
           195                 200                 205
   Leu Ala Gln Gln Arg Cys Asn Leu Asp Asp Thr Trp Glu Gly Lys Ile
       210                 215                 220
   Tyr Arg Val Leu Ala Gly Asn Pro Ala Lys His Asp Leu Asp Ile Lys
   225                 230                 235                 240
   Pro Thr Val Ile Ser His Arg Leu His Phe Pro Glu Gly Gly Ser Leu
                   245                 250                 255
   Ala Ala Leu Thr Ala His Gln Ala Cys His Leu Pro Leu Glu Thr Phe
               260                 265                 270
   Thr Arg His Arg Gln Pro Arg Gly Trp Glu Gln Leu Glu Gln Cys Gly
           275                 280                 285
   Tyr Pro Val Gln Arg Leu Val Ala Leu Tyr Leu Ala Ala Arg Leu Ser
       290                 295                 300
   Trp Asn Gln Val Asp Gln Val Ile Arg
   305                 310
<210> 6
   <211> 112
   <212> PRT
   <213> Pseudomonas aeruginosa
<400> 6
   Gly Gly Ser Leu Ala Ala Leu Thr Ala His Gln Ala Cys His Leu Pro
   1               5                   10                  15
   Leu Glu Thr Phe Thr Arg His Arg Gln Pro Arg Gly Trp Glu Gln Leu
               20                  25                  30
   Glu Gln Cys Gly Tyr Pro Val Gln Arg Leu Val Ala Leu Tyr Leu Ala
           35                  40                  45
   Ala Arg Leu Ser Trp Asn Gln Val Asp Gln Val Ile Arg Asn Ala Leu
   50 55 60
   Ala Ser Pro Gly Ser Gly Gly Asp Leu Gly Glu Ala Ile Arg Glu Gln
   65 70 75 80
   Pro Glu Gln Ala Arg Leu Ala Leu Thr Leu Ala Ala Ala Glu Ser Glu
   85 90 95
   Arg Phe Val Arg Gln Gly Thr Gly Asn Asp Glu Ala Gly Ala Ala Asn
   100 105 110
<210> 7
   <211> 407
   <212> PRT
   <213> Pseudomonas aeruginosa
<400> 7
   Ala Glu Glu Ala Phe Asp Leu Trp Asn Glu Cys Ala Lys Ala Cys Val
   1 5 10 15
   Leu Asp Leu Lys Asp Gly Val Arg Ser Ser Arg Met Ser Val Asp Pro
   20 25 30
   Ala Ile Ala Asp Thr Asn Gly Gln Gly Val Leu His Tyr Ser Met Val
   35 40 45
   Leu Glu Gly Gly Asn Asp Ala Leu Lys Leu Ala Ile Asp Asn Ala Leu
   50 55 60
   Ser Ile Thr Ser Asp Gly Leu Thr Ile Arg Leu Glu Gly Gly Val Glu
   65 70 75 80
   Pro Asn Lys Pro Val Arg Tyr Ser Tyr Thr Arg Gln Ala Arg Gly Ser
   85 90 95
   Trp Ser Leu Asn Trp Leu Val Pro Ile Gly His Glu Lys Pro Ser Asn
   100 105 110
   Ile Lys Val Phe Ile His Glu Leu Asn Ala Gly Asn Gln Leu Ser His
   115 120 125
   Met Ser Pro Ile Tyr Thr Ile Glu Met Gly Asp Glu Leu Leu Ala Lys
   130 135 140
   Leu Ala Arg Asp Ala Thr Phe Phe Val Arg Ala His Glu Ser Asn Glu
   145 150 155 160
   Met Gln Pro Thr Leu Ala Ile Ser His Ala Gly Val Ser Val Val Met
                   165                 170                 175
   Ala Gln Thr Gln Pro Arg Arg Glu Lys Arg Trp Ser Glu Trp Ala Ser
               180                 185                 190
   Gly Lys Val Leu Cys Leu Leu Asp Pro Leu Asp Gly Val Tyr Asn Tyr
           195                 200                 205
   Leu Ala Gln Gln Arg Cys Asn Leu Asp Asp Thr Trp Glu Gly Lys Ile
       210                 215                 220
   Tyr Arg Val Leu Ala Gly Asn Pro Ala Lys His Asp Leu Asp Ile Lys
   225                 230                 235                 240
   Pro Thr Val Ile Ser His Arg Leu His Phe Pro Glu Gly Gly Ser Leu
                   245                 250                 255
   Ala Ala Leu Thr Ala His Gln Ala Cys His Leu Pro Leu Glu Thr Phe
               260                 265                 270
   Thr Arg His Arg Gln Pro Arg Gly Trp Glu Gln Leu Glu Gln Cys Gly
           275                 280                 285
   Tyr Pro Val Gln Arg Leu Val Ala Leu Tyr Leu Ala Ala Arg Leu Ser
       290                 295                 300
   Trp Asn Gln Val Asp Gln Val Ile Arg Asn Ala Leu Ala Ser Pro Gly
   305                 310                 315                 320
   Ser Gly Gly Asp Leu Gly Glu Ala Ile Arg Glu Gln Pro Glu Gln Ala
                   325                 330                 335
   Arg Leu Ala Leu Thr Leu Ala Ala Ala Glu Ser Glu Arg Phe Val Arg
               340                 345                 350
   Gln Gly Thr Gly Asn Asp Glu Ala Gly Ala Ala Asn Ala Asp Val Val
           355                 360                 365
   Ser Leu Thr Cys Pro Val Ala Ala Gly Glu Cys Ala Gly Pro Ala Asp
       370                 375                 380
   Ser Gly Asp Ala Leu Leu Glu Arg Asn Tyr Pro Thr Gly Ala Glu Phe
   385                 390                 395                 400
   Leu Gly Asp Gly Gly Asp Val
                   405
<210> 8
   <211> 104
   <212> PRT
   <213> Homo sapiens
<400> 8
   Ala Glu Phe Glu Glu Pro Arg Val Ile Asp Leu Trp Asp Leu Ala Gln
   1               5                   10                  15
   Ser Ala Asn Leu Thr Asp Lys Glu Leu Glu Ala Phe Arg Glu Glu Leu
               20                  25                  30
   Lys His Phe Glu Ala Lys Ile Glu Lys His Asn His Tyr Gln Lys Gln
           35                  40                  45
   Leu Glu Ile Ala His Glu Lys Leu Arg His Ala Glu Ser Val Gly Asp
       50                  55                  60
   Gly Glu Arg Val Ser Arg Ser Arg Glu Lys His Ala Leu Leu Glu Gly
   65                  70                  75                  80
   Arg Thr Lys Glu Leu Gly Tyr Thr Val Lys Lys His Leu Gln Asp Leu
                   85                  90                  95
   Ser Gly Arg Ile Ser Arg Ala Arg
               100
<210> 9
   <211> 153
   <212> PRT
   <213> Artificial Sequence
<220>
   <223> A2M Minimum
<400> 9
   Val Tyr Leu Gln Thr Ser Leu Lys Tyr Asn Ile Leu Pro Glu Lys Glu
   1               5                   10                  15
   Glu Phe Pro Phe Ala Leu Gly Val Gln Thr Leu Pro Gln Thr Cys Asp
               20                  25                  30
   Glu Pro Lys Ala His Thr Ser Phe Gln Ile Ser Leu Ser Val Ser Tyr
           35                  40                  45
   Thr Gly Ser Arg Ser Ala Ser Asn Met Ala Ile Val Asp Val Lys Met
       50                  55                  60
   Val Ser Gly Phe Ile Pro Leu Lys Pro Thr Val Lys Met Leu Glu Arg
   65                  70                  75                  80
   Ser Asn His Val Ser Arg Thr Glu Val Ser Ser Asn His Val Leu Ile
                   85                  90                  95
   Tyr Leu Asp Lys Val Ser Asn Gln Thr Leu Ser Leu Phe Phe Thr Val
               100                 105                 110
   Leu Gln Asp Val Pro Val Arg Asp Leu Lys Pro Ala Ile Val Lys Val
           115                 120                 125
   Tyr Asp Tyr Tyr Glu Thr Asp Glu Phe Ala Ile Ala Glu Tyr Asn Ala
       130                 135                 140
   Pro Cys Ser Lys Asp Leu Gly Asn Ala
   145                 150
<210> 10
   <211> 24
   <212> PRT
   <213> Artificial Sequence
<220>
   <223> HIV-Tat Minimum
<400> 10
   Arg Gly Asp Pro Thr Gly Gln Glu Glu Ser Lys Glu Lys Val Glu Lys
   1               5                   10                  15
   Glu Thr Val Val Asp Pro Val Thr
               20
<210> 11
   <211> 641
   <212> PRT
   <213> Artificial Sequence
<220>
   <223> HSPs Minimum
<400> 11 Asp
<210> 12
   <211> 15
   <212> PRT
   <213> Artificial Sequence
<220>
   <223> NESK
<400> 12
   Leu Gln Lys Lys Leu Glu Glu Leu Glu Leu Ala Lys Asp Glu Leu
   1 5 10 15
<210> 13
   <211> 11
   <212> PRT
   <213> Artificial Sequence
<220>
   <223> NES consensus sequence
<220>
   <221> misc_feature
   <222> (2)..(3)
   <223> Xaa can be any naturally occurring amino acid
<220>
   <221> misc_feature
   <222> (6)..(7)
   <223> Xaa can be any naturally occurring amino acid
<220>
   <221> misc_feature
   <222> (9)..(9)
   <223> Xaa can be any naturally occurring amino acid
<220>
   <221> misc_feature
   <222> (11)..(11)
   <223> Xaa can be any naturally occurring amino acid
<400> 13
   Leu Xaa Xaa Lys Leu Xaa Xaa Leu Xaa Leu Xaa
   1               5                   10
<210> 14
   <211> 11
   <212> PRT
   <213> Artificial Sequence
<220>
   <223> NES
<400> 14
   Leu Gln Lys Lys Leu Glu Glu Leu Glu Leu Ala
   1               5                   10
<210> 15
   <211> 4
   <212> PRT
   <213> Artificial Sequence
<220>
   <223> ER retention sequence
<400> 15
   Lys Asp Glu Leu
   1
<210> 16
   <211> 12
   <212> PRT
   <213> Artificial Sequence
<220>
   <223> K3
<400> 16
   Lys Asp Glu Leu Lys Asp Glu Leu Lys Asp Glu Leu
   1               5                   10
<210> 17
   <211> 12
   <212> PRT
   <213> Artificial Sequence
<220>
   <223> K3
<400> 17
   Lys Lys Asp Leu Arg Asp Glu Leu Lys Asp Glu Leu
   1 5 10
<210> 18
   <211> 13
   <212> PRT
   <213> Artificial Sequence
<220>
   <223> K3
<400> 18
   Lys Lys Asp Glu Leu Arg Asp Glu Leu Lys Asp Glu Leu
   1 5 10
<210> 19
   <211> 13
   <212> PRT
   <213> Artificial Sequence
<220>
   <223> K3
<400> 19
   Lys Lys Asp Glu Leu Arg Val Glu Leu Lys Asp Glu Leu
   1 5 10
<210> 20
   <211> 192
   <212> PRT
   <213> Artificial Sequence
<220>
   <223> truncated PCV2 ORF2
<400> 20
   Asn Gly Ile Phe Asn Thr Arg Leu Ser Arg Thr Phe Gly Tyr Thr Ile
   1 5 10 15
   Lys Arg Thr Thr Val Lys Thr Pro Ser Trp Ala Val Asp Met Met Arg
   20 25 30
   Phe Asn Ile Asn Asp Phe Leu Pro Pro Gly Gly Gly Ser Asn Pro Arg
   35 40 45
   Ser Val Pro Phe Glu Tyr Tyr Ser Ile Ser Lys Val Lys Val Glu Phe
   50 55 60
   Trp Pro Cys Ser Pro Ile Thr Gln Gly Asp Ser Gly Val Gly Ser Ser
   65 70 75 80
   Ala Val Ile Leu Asp Asp Asn Phe Val Thr Lys Ala Thr Ala Leu Thr
                   85                  90                  95
   Tyr Asp Pro Tyr Val Asn Tyr Ser Ser Arg His Thr Ile Thr Gln Pro
               100                 105                 110
   Phe Ser Tyr His Ser Arg Tyr Phe Thr Pro Lys Pro Val Leu Asp Ser
           115                 120                 125
   Thr Ile Asp Tyr Phe Gln Pro Asn Asn Lys Arg Asn Gln Leu Trp Leu
       130                 135                 140
   Arg Leu Gln Thr Ala Gly Asn Val Asp His Val Gly Leu Gly Thr Ala
   145                 150                 155                 160
   Phe Glu Asn Ser Ile Tyr Asp Gln Glu Tyr Asn Ile Arg Val Thr Met
                   165                 170                 175
   Tyr Val Gln Phe Arg Glu Phe Asn Leu Lys Asp Pro Pro Leu Asn Pro
               180                 185                 190
<210> 21
   <211> 328
   <212> PRT
   <213> Artificial Sequence
<220>
   <223> truncated CSFV E2
<400> 21
   Arg Leu Ser Cys Lys Glu Asp His Arg Tyr Ala Ile Ser Ser Thr Asn
   1               5                   10                  15
   Glu Ile Gly Pro Leu Gly Ala Glu Gly Leu Thr Thr Thr Trp Lys Glu
               20                  25                  30
   Tyr Ser His Gly Leu Gln Leu Asp Asp Gly Thr Val Arg Ala Ile Cys
           35                  40                  45
   Ile Ala Gly Ser Phe Lys Val Thr Ala Leu Asn Val Val Ser Arg Arg
       50                  55                  60
   Tyr Leu Ala Ser Leu His Lys Arg Ala Leu Pro Thr Ser Val Thr Phe
   65                  70                  75                  80
   Glu Leu Leu Phe Asp Gly Thr Ser Pro Ala Ile Glu Glu Met Gly Glu
                   85                  90                  95
   Asp Phe Gly Phe Gly Leu Cys Pro Phe Asp Thr Thr Pro Val Val Lys
               100                 105                 110
   Gly Lys Tyr Asn Thr Thr Leu Leu Asn Gly Ser Ala Phe Tyr Leu Val
           115                 120                 125
   Cys Pro Ile Gly Trp Thr Gly Val Ile Glu Cys Thr Ala Val Ser Pro
       130                 135                 140
   Thr Thr Leu Arg Thr Glu Val Val Lys Thr Phe Lys Arg Glu Lys Pro
   145                 150                 155                 160
   Phe Pro His Arg Ala Asp Cys Val Thr Thr Ile Val Glu Lys Glu Asp
                   165                 170                 175
   Leu Phe His Cys Lys Leu Gly Gly Asn Trp Thr Cys Val Lys Gly Asn
               180                 185                 190
   Pro Val Thr Tyr Thr Gly Gly Gln Val Lys Gln Cys Arg Trp Cys Gly
           195                 200                 205
   Phe Asp Phe Lys Glu Pro Asp Gly Leu Pro His Tyr Pro Ile Gly Lys
       210                 215                 220
   Cys Ile Leu Ala Asn Glu Thr Gly Tyr Arg Val Val Asp Ser Thr Asp
   225                 230                 235                 240
   Cys Asn Arg Asp Gly Val Val Ile Ser Thr Glu Gly Glu His Glu Cys
                   245                 250                 255
   Leu Ile Gly Asn Thr Thr Val Lys Val His Ala Leu Asp Gly Arg Leu
               260                 265                 270
   Ala Pro Met Pro Cys Arg Pro Lys Glu Ile Val Ser Ser Ala Gly Pro
           275                 280                 285
   Val Arg Lys Thr Ser Cys Thr Phe Asn Tyr Thr Lys Thr Leu Arg Asn
       290                 295                 300
   Lys Tyr Tyr Glu Pro Arg Asp Ser Tyr Phe Gln Gln Tyr Met Leu Lys
   305                 310                 315                 320
   Gly Glu Tyr Gln Tyr Trp Phe Asp
                   325
<210> 22
   <211> 50
   <212> PRT
   <213> Artificial Sequence
<220>
   <223> FMDV VP1 peptide
<400> 22
   Ala Thr Val Tyr Asn Gly Ser Ser Lys Tyr Gly Asp Thr Ser Thr Ser
   1               5                   10                  15
   Asn Val Arg Gly Asp Leu Gln Val Leu Ala Gln Lys Ala Glu Arg Thr
               20                  25                  30
   Leu Pro Thr Ser Phe Asn Phe Gly Ala Ile Lys Ala Thr Arg Val Thr
           35                  40                  45
   Glu Leu
       50
<210> 23
   <211> 15
   <212> PRT
   <213> Artificial Sequence
<220>
   <223> FMDV 3A peptide
<400> 23
   Ala Ala Ile Glu Phe Phe Glu Gly Met Val His Asp Ser Ile Lys
   1               5                   10                  15
<210> 24
   <211> 65
   <212> PRT
   <213> Artificial Sequence
<220>
   <223> FMDV VP1-3A peptide
<400> 24 Lys
   65
<210> 25
   <211> 18
   <212> PRT
   <213> Artificial Sequence
<220>
   <223> NDV F peptide
<400> 25 Leu Glu
<210> 26
   <211> 11
   <212> PRT
   <213> Artificial Sequence
<220>
   <223> NDV HN peptide
<400> 26
   Pro Asp Glu Gln Asp Tyr Gln Ile Arg Met Ala
   1               5                   10
<210> 27
   <211> 29
   <212> PRT
   <213> Artificial Sequence
<220>
   <223> NDV FHN peptide
<400> 27
   Leu Leu Pro Asn Met Pro Lys Asp Lys Glu Gly Cys Ala Lys Ala Pro
   1 5 10 15
   Leu Glu Pro Asp Glu Gln Asp Tyr Gln Ile Arg Met Ala
   20 25
<210> 28
   <211> 98
   <212> PRT
   <213> Human papillomavirus type 16
<400> 28 Lys Pro
<210> 29
   <211> 613
   <212> PRT
   <213> Pseudomonas aeruginosa
<400> 29
   Ala Glu Glu Ala Phe Asp Leu Trp Asn Glu Cys Ala Lys Ala Cys Val
   1               5                   10                  15
   Leu Asp Leu Lys Asp Gly Val Arg Ser Ser Arg Met Ser Val Asp Pro
               20                  25                  30
   Ala Ile Ala Asp Thr Asn Gly Gln Gly Val Leu His Tyr Ser Met Val
           35                  40                  45
   Leu Glu Gly Gly Asn Asp Ala Leu Lys Leu Ala Ile Asp Asn Ala Leu
       50                  55                  60
   Ser Ile Thr Ser Asp Gly Leu Thr Ile Arg Leu Glu Gly Gly Val Glu
   65                  70                  75                  80
   Pro Asn Lys Pro Val Arg Tyr Ser Tyr Thr Arg Gln Ala Arg Gly Ser
                   85                  90                  95
   Trp Ser Leu Asn Trp Leu Val Pro Ile Gly His Glu Lys Pro Ser Asn
               100                 105                 110
   Ile Lys Val Phe Ile His Glu Leu Asn Ala Gly Asn Gln Leu Ser His
           115                 120                 125
   Met Ser Pro Ile Tyr Thr Ile Glu Met Gly Asp Glu Leu Leu Ala Lys
       130                 135                 140
   Leu Ala Arg Asp Ala Thr Phe Phe Val Arg Ala His Glu Ser Asn Glu
   145                 150                 155                 160
   Met Gln Pro Thr Leu Ala Ile Ser His Ala Gly Val Ser Val Val Met
                   165                 170                 175
   Ala Gln Thr Gln Pro Arg Arg Glu Lys Arg Trp Ser Glu Trp Ala Ser
               180                 185                 190
   Gly Lys Val Leu Cys Leu Leu Asp Pro Leu Asp Gly Val Tyr Asn Tyr
           195                 200                 205
   Leu Ala Gln Gln Arg Cys Asn Leu Asp Asp Thr Trp Glu Gly Lys Ile
       210                 215                 220
   Tyr Arg Val Leu Ala Gly Asn Pro Ala Lys His Asp Leu Asp Ile Lys
   225                 230                 235                 240
   Pro Thr Val Ile Ser His Arg Leu His Phe Pro Glu Gly Gly Ser Leu
                   245                 250                 255
   Ala Ala Leu Thr Ala His Gln Ala Cys His Leu Pro Leu Glu Thr Phe
               260                 265                 270
   Thr Arg His Arg Gln Pro Arg Gly Trp Glu Gln Leu Glu Gln Cys Gly
           275                 280                 285
   Tyr Pro Val Gln Arg Leu Val Ala Leu Tyr Leu Ala Ala Arg Leu Ser
       290                 295                 300
   Trp Asn Gln Val Asp Gln Val Ile Arg Asn Ala Leu Ala Ser Pro Gly
   305                 310                 315                 320
   Ser Gly Gly Asp Leu Gly Glu Ala Ile Arg Glu Gln Pro Glu Gln Ala
                   325                 330                 335
   Arg Leu Ala Leu Thr Leu Ala Ala Ala Glu Ser Glu Arg Phe Val Arg
               340                 345                 350
   Gln Gly Thr Gly Asn Asp Glu Ala Gly Ala Ala Asn Ala Asp Val Val
           355                 360                 365
   Ser Leu Thr Cys Pro Val Ala Ala Gly Glu Cys Ala Gly Pro Ala Asp
       370                 375                 380
   Ser Gly Asp Ala Leu Leu Glu Arg Asn Tyr Pro Thr Gly Ala Glu Phe
   385                 390                 395                 400
   Leu Gly Asp Gly Gly Asp Val Ser Phe Ser Thr Arg Gly Thr Gln Asn
                   405                 410                 415
   Trp Thr Val Glu Arg Leu Leu Gln Ala His Arg Gln Leu Glu Glu Arg
               420                 425                 430
   Gly Tyr Val Phe Val Gly Tyr His Gly Thr Phe Leu Glu Ala Ala Gln
           435                 440                 445
   Ser Ile Val Phe Gly Gly Val Arg Ala Arg Ser Gln Asp Leu Asp Ala
       450                 455                 460
   Ile Trp Arg Gly Phe Tyr Ile Ala Gly Asp Pro Ala Leu Ala Tyr Gly
   465                 470                 475                 480
   Tyr Ala Gln Asp Gln Glu Pro Asp Ala Arg Gly Arg Ile Arg Asn Gly
                   485                 490                 495
   Ala Leu Leu Arg Val Tyr Val Pro Arg Ser Ser Leu Pro Gly Phe Tyr
               500                 505                 510
   Arg Thr Ser Leu Thr Leu Ala Ala Pro Glu Ala Ala Gly Glu Val Glu
           515                 520                 525
   Arg Leu Ile Gly His Pro Leu Pro Leu Arg Leu Asp Ala Ile Thr Gly
       530                 535                 540
   Pro Glu Glu Glu Gly Gly Arg Leu Glu Thr Ile Leu Gly Trp Pro Leu
   545                 550                 555                 560
   Ala Glu Arg Thr Val Val Ile Pro Ser Ala Ile Pro Thr Asp Pro Arg
                   565                 570                 575
   Asn Val Gly Gly Asp Leu Asp Pro Ser Ser Ile Pro Asp Lys Glu Gln
               580                 585                 590
   Ala Ile Ser Ala Leu Pro Asp Tyr Ala Ser Gln Pro Gly Lys Pro Pro
           595                 600                 605
   Arg Glu Asp Leu Lys
       610